# EUROPÄISCHE PATENTSCHRIFT

(11) **EP 0 962 441 B1**
(45) Veröffentlichungstag und Bekanntmachung des Hinweises auf die Patenterteilung: **13.11.2002**
(21) Anmeldenummer: 99110538.8
(22) Anmeldetag: 01.06.1999
(51) Int. Cl.: C07C 49/553, C07C 47/445, C07C 45/69, C07C 13/32, C11B 9/00, A61K 7/46

(54) **Bicyclische Aldehyde und Ketone**
Bicyclic aldehydes and ketones
Aldéhydes et cétones bicycliques

(30) Priorität: 03.06.1998 CH 120298
(43) Veröffentlichungstag der Anmeldung: 08.12.1999
(73) Patentinhaber: Givaudan SA, 1214 Vernier-Genève (CH)
(72) Erfinder: Frater, Georg, 8400 Winterthur (CH); Kraft, Philip, 8600 Dübendorf (CH)
(74) Vertreter: Patentanwälte Schaad, Balass, Menzl & Partner AG

(56) Entgegenhaltungen:
- US-A- 4 522 748
- US-A- 5 077 275
- KRAFT P: "Design and synthesis of violet odorants with bicyclo[6.4.0]dodecene and bicyclo[5.4.0]undecene skeletons" SYNTHESIS (SYNTBF,00397881);1999; (4); PP.695-703, XP002111306 Givaudan Roure Fragrance Res. Ltd.;Duebendorf; CH-8600; Switz. (CH)
- POSNER G H ET AL: "Sequential Michael-Michael-ring closure reactions for 3-different-component, one-pot, 2+2+2 construction of acylcyclohexenes and an acylcyclohexanol" TETRAHEDRON LETT. (TELEAY,00404039);1986; VOL.27 (6); PP.659-62, XP002111307 Johns Hopkins Univ.;Dep. Chem.; Baltimore; 21218; MD; USA (US)
- ALPOIM M C M D C ET AL: "Ring-opening reactions of vinylcyclopropanes with p-toluenesulfonyl iodide" TETRAHEDRON LETT. (TELEAY,00404039);1988; VOL.29 (33); PP.4173-6, XP002111308 Imp. Coll. Sci. Technol.;Dep. Chem.; South Kensington/London; SW7 2AY; UK (GB)

## Beschreibung

Die Erfindung betrifft Aldehyde und Ketone mit Bicyclo[6.4.0]dodec-1(8)-en-10-yl-, Bicyclo[5.4.0]undec-1(7)-en-9-yl-, Bicyclo[6.4.0]dodec-1(12)-en-10-yloder Bicyclo[5.4.0]undec-1(11)-en-9-yl-Skelett, insbesondere 1-{Bicyclo(6.4.0]dodec-1(8)-en-10-yl}ethan-1-one, 1-{Bicyclo[6.4.0]dodec-1(8)-en-10-yl}methanale, 1-{Bicyclo[5.4.0]undec-1(7)-en-9-yl}ethan-1-one, 1-{Bicyclo[5.4.0]undec-1(7)-en-9-yl}methanale, 1-{Bicyclo[6.4.0]dodec-1(12)-en-10-yl}ethan-1-one, 1-{Bicyclo[6.4.0]dodec-1(12)-en-10-yl}methanale, 1-{Bicyclo[5.4.0]undec-1(11)-en-9-yl}ethan-1-one und 1-{Bicyclo[5.4.0]undec-1(12)-en-9-yl}methanale, die am sechsgliedrigen Ring methyl- oder ethylsubstituiert sein können, sowie ihre Verwendung als Riechstoffe.

Jonone und ihre linearen (etwa Raldeine® oder Timberol®) wie cyclischen Derivate (etwa Iso E super®) spielen eine zentrale Rolle in der Parfümerie. Vor allem seitdem in den letzten zehn Jahren die klassischen Parfüms aus Kopf-, Herz- und Basisnoten mehr und mehr von monolithischen Kreationen abgelöst wurden, die um einen Hauptakkord aus relativ wenigen Duftstoffen aufgebaut sind, von denen einige bis zu 25% und mehr in einer Komposition erreichen können [R. R. Calkin, J. S. Jellinek, Perfumery - Practice and Principles, Wiley, New York 1994, 138-140]. Jonone und ihre Derivate sind bevorzugte Duftstoffe für die Komposition solcher Akkorde und Parfüms, da sie sich harmonisch mit einer Vielzahl von Duftbausteinen verbinden, den Kompositionen Transparenz und Wärme verleihen und ihre weichen, floral-holzigen Noten selbst in hoher Konzentration angenehm bleiben. Andererseits sind Jonone und Jononderivate eine relativ alte Riechstoffklasse, und obwohl sie ein ganzes Spektrum von frisch floralen bis hin zu stark holzig-ambraartigen Facetten abdecken, besteht dennoch ein großer Bedarf an neuen Verbindungen ähnlicher Tonalität jedoch mit neuem, eigenständigem Duftcharakter, um neue originelle Akkorde zu kreieren um die herum sich neue Trendparfüms (etwa die sogenannten "Sheer Orientals") komponieren lassen.

In Aldehyden und Ketonen mit den oben näher bezeichneten Bicycloskeletten wurde nun eine Klasse von Verbindungen gefunden, die das holzig-florale Duftspektrum von Jononen und Jononderivaten um neue Facetten bereichert und damit obgenannten Ansprüchen in vorteilhafter Weise genügt. Diese neue Verbindungsklasse wird durch die Formel I 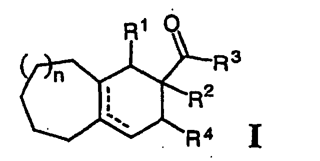
wiedergegeben, worin n = 1, 2
und R¹, R⁴ = H oder CH₃
und R², R³ = H, CH₃ oder CH₂CH₃
darstellen mit Ausnahme der Verbindung der Formel I, worin
n=1,
R¹ = R² = R⁴, = H, und
R³ = CH₂CH₃
ist und die gestrichelte Linie eine Doppelbindung zwischen den beiden Brückenkopfatomen oder zwischen dem Brückenkopfatom 1 und dem Ringatom 11 bzw. 12 bedeutet.

Die Verbindungen der Formel I sind neu. Formel I umfasst demgemäß Aldehyde sowie Methyl- und Ethyl- Ketone mit Bicyclo[6.4.0]dodec-1(8)-en-10-yl- oder Bicyclo[5.4.0]undec-1(7)-en-9-yl- bzw. Bicyclo[6.4.0]dodec-1(12)-en-10-yl- oder Bicyclo[5.4.0]undec-1(11)-en-9-yl- Resten, welche am Cyclohexen-ring Methyl- oder Ethylsubstituenten tragen können. Die folgenden Verbindungen 1-14 sind bevorzugte Beispiele dieser neuen Stoffklasse:

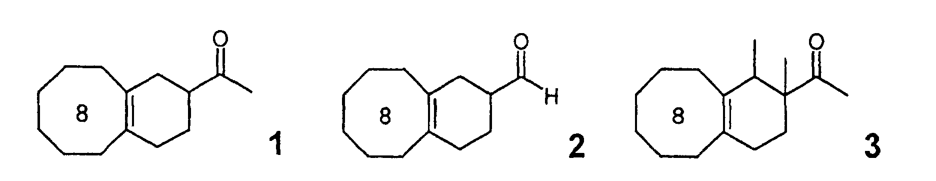

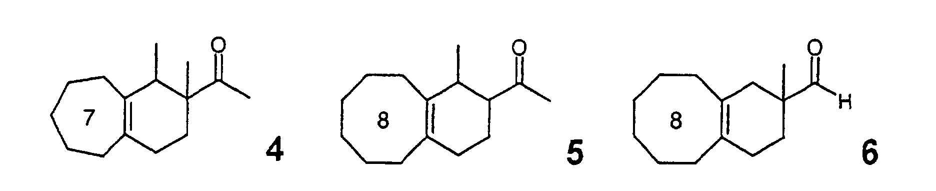

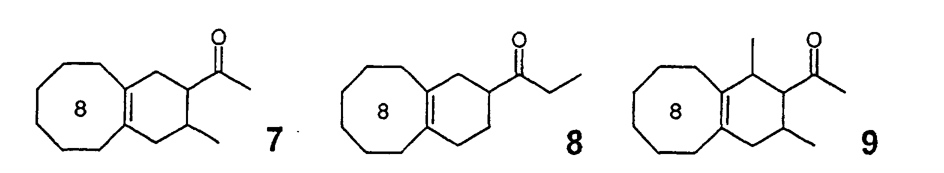

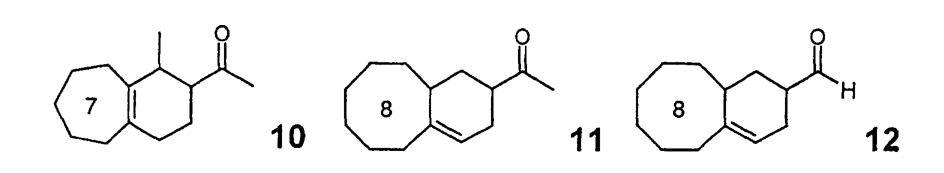

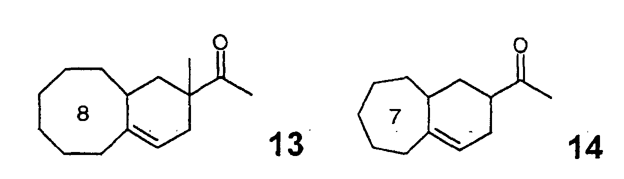

Die Verbindungen der Formel I besitzen holzig-blumige Noten, die an Jonone erinnern, aber zusätzlich aldehydige, maritime, ambra- und tabakartige, teilweise auch fruchtig-frische, melonenartige, oder balsamische Nebennoten besitzen. In einigen Fällen, z.B. bei den Verbindungen 3 und 4, dominiert der holzige Charakter stark, und der Gesamtgeruchseindruck ist holzig-ambraartig bis trocken-weihrauchartig, an Iso E super®, aber auch an Vetiveröl erinnernd. In vorteilhafter Weise parfümistisch interessant sind neben diesen im Rahmen der Erfindung herausragenden Verbindungen 3 und 4 vor allem die Aldehyde 2 und 6, gefolgt von den R¹-methylsubstituierten Verbindungen 5 und 10. Die Verbindungen der Formel I, und somit insbesondere die Verbindungen 1-13, sollen erfindungsgemäss alle möglichen Stereo- und Diastereomere der betreffenden Verbindungen umfassen. Diese erfindungsgemässen Verbindungen unterscheiden sich in ihrer Intensität und ihrem Geruch teilweise stark, aufgrund ihres Geruchs sind sie aber in jedem Fall parfümistisch einsetzbar. In den nachfolgenden Beispielen wird das jeweils geruchsintensivste und somit besonders interessante Diastereomere beschrieben und näher charakterisiert.

Die Verbindungen der Formel I harmonieren mit einer Vielzahl natürlicher und/oder synthetischer Rohstoffe, die häufig in Riechstoffkompositionen eingesetzt werden. Besonders in den Duftrichtungen Floral und Chypre lassen sich interessante Akkorde und Parfüms um die Verbindungen der Formel I aufbauen. Hervorragend eignen sich die Verbindungen der Formel I aber auch für monolithisch aufgebaute Parfüms orientalischer-ambrierter Duftrichtungen. Ferner lassen sich auch ungewöhnliche, neue betont holzige Kompositionen wie etwa vom Typ "Féminité du Bois" (Shiseido 1992) sehr vorteilhaft mit den Verbindungen der Formel I aufbauen.

Die erfindungsgemässe Verwendung der Verbindungen der Formel I ist aber weder auf diese Parfümtypen beschränkt, noch auf spezielle Duftrichtungen, Riechstoffderivate oder chemische Substanzklassen. Beispiele für besonders gut harmonierende Substanzklassen sind:
- Ätherische Öle und Extrakte, z. B. Bayöl, Bergamottöl, Cedernholzöl, Geraniumöl, Guajakholzöl, Patchouliöl, Petitgrainöl, Rosenöl, Rosenholzöl, Vetiveröl, Ylang-
   Yang-Öl;
- Alkohole, z. B. Citronellol, Dimetol®, Ebanol®, cis-3-Hexenol, Geraniol, Linalool, Peonile®, Phenoxanol, Rosalva®,
   Rosaphen®,
   Sandalore®, Zimtalkohol;
- Aldehyde und Ketone, z. B. alpha-Amylzimtaldehyd, Cashmeran®, beta-Damascenon, Dupical®, Florhydral®, Frambinon®, Givescone®, Hedion®, Hydroxycitronellal,
   Lilial®,
   Vanillin;
- Ether und Acetale, z. B. Ambrox®, Calone®, Galaxolide®, Magnolan®, Rhubafuran®, Rosenoxid, Spirambrene®;
- Ester und Lactone, z. B. Agrumex®, Benzylacetat, Benzylsalicylat, Citronellylacetat, delta- und gamma-Decalacton, gamma-Undecalacton;
- Makrocyclen, z. B. Ambrettolid®, Ambreton®, Muscon, Musk R-1, Thibetolid®, Trimofix O®;
- Heterocyclen, z. B. Indol, Skatol.

Die Synthese der Verbindungen gemäss Formel I kann einerseits, wie in den Beispielen 1 und 11 exemplarisch ausgeführt, durch Diels-Alder-Reaktion der entsprechenden Bis(methylen)cycloalkane, der Ethylidenmethylencycloalkane oder der 1-Vinylcycloalkene, zugänglich etwa nach der Vorschrift von J. W. van Straten, J. J. van Norden, T. A. M. van Schaik, G.T. Franke, W. H. de Wolf, F. Bickelhaupt [Recl. Trav. Chim. Pays-Bas 1978, 97, 105] bzw. von W. Herz und R.-R. Juo [J. Org. Chem. 1985,50,618], erfolgen. Erfindungsgemäss wurde nun andererseits für die R¹-methylsubstituierten Verbindungen mit brückenkopfständiger Doppelbindung (I') der Formel I ein neuer Weg über Vinylcyclopropane (V) gefunden, der in Beispiel 6 exemplarisch ausgeführt wird. Dabei werden 4-Methylenspiro[2.7]decan bzw. 4-Methylenspiro[2.6]nonan, die aus Spiro[2.7]decan-4-on respektive Spiro[2.6]nonan-4-on durch Methylenierung etwa über eine Wittig-Reaktion zugänglich sind, mit einem Katalysator, vorzugsweise dem Wilkinson Katalysator, in einem hochsiedenden, relativ unpolaren Lösungsmittel wie z.B. Toluol oder Xylol mehrere Stunden bis einige Tage unter Rückfluss erhitzt. Als Hauptprodukt dieser neuen Reaktion werden dann regioselektiv und in einigen Fällen, wie im Beispiel 6, auch Z- diastereoselektiv die Verbindungen I' isoliert. Da die Ausgangsmaterialien Spiro[2.7]decan-4-on und Spiro[2.6]nonan-4-on nach einer Vorschrift von S. M. Ruder, R. C. Ronald [Tetrahedron Lett. 1984, 25, 5501] einfach zugänglich sind, stellt dieser Weg eine attraktive Alternative zur Synthese der Verbindungen I' über exocyclische Diene dar: 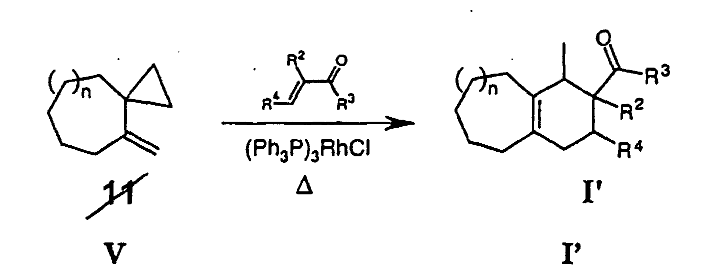 worin
n = 1, 2 and R⁴ = H oder CH₃
sowie R², R³ = H, CH₃ oder CH₂CH₃
darstellen.

Ein weiterer Vorteil dieses Verfahrens ist, daß das Zwischenprodukt V mit n = 1 selbst einen neuen, interessant frischen grün-minzigen, etwas holzigfruchtigen Riechstoff darstellt, der ebenfalls in der Parfümerie eingesetzt werden kann.

### Beispiel 1

### 1-{Bicyclo[6.4.0]dodec-1'(8')-en-10'-yl}ethan-1-on (1)

Das Ausgangsmaterial, 1,2-Bis(methylen)cyclooctan, wurde nach der Vorschrift von J. W. van Straten, J. J. van Norden, T. A. M. van Schaik, G. T. Franke, W. H. de Wolf, F. Bickelhaupt [Recl. Trav. Chim. Pays-Bas 1978, 97, 105] hergestellt.

Zu einer Lösung von 2.00 g (14.7 mmol) 1,2-Bis(methylen)cyclooctan in 20 ml trockenem Toluol wurde unter Rühren 1.46 ml (17.8 mmol) But-3-en-2-on gegeben und das Reaktionsgemisch auf 0 °C abgekühlt. Bei dieser Temp. wurden unter Stickstoff 160 mg (1.20 mmol, 7 mol%) Aluminimtrichlorid hinzugegeben. Nach 5 min. Rühren bei 0 °C entfernte man die Kühlung, liess die Reaktionsmischung 60 h unter Stickstoff bei Raumtemp. rühren und goss darauf die Mischung in 400 ml tert-Butylmethylether / Wasser (1:1). Die organische Phase wurde abgetrennt und die wässrige Phase dreimal mit 200 ml tert-Butylmethylether extrahiert. Die organischen Phasen wurden vereinigt, über Natriumsulfat getrocknet und am Rotationsverdampfer eingeengt. Nach Flashchromatographie (n-Pentane / tert-Butylmethylether, 20:1, *R*_{f} = 0.58) an Kieselgel erhielt man 2.54 g (84 %) 1-{Bicyclo[6.4.0]dodec-1'(8')-en-10'-yl}ethan-1-on (1) als farblose Flüssigkeit.

Geruch: Holzig-blumig nach β-Jonon, fruchtig-frisch, leicht aldehydig und ambraartig. - IR (Film): ν = 1711 cm⁻¹ (ν C=O). - ¹H-NMR (CDCl₃): δ =1.35-1.57 (m, 10H, 3'-H₂-6'-H₂, 11'-H₂), 2.18 (s, 3H, 2-H₃), 1.93-2.14 (m, 8H, 2'-,7'-,9'-,12'-H₂), 2.55 (dddd, J 11.5, 10.1, 5.3, 2.8 Hz, 1H, 10'-H). - ¹³C-NMR (CDCl₃): δ = 25.29 (t, C-11'), 26.47 / 26.48 (t, C-3',-6'), 27.88 (q, C-2), 28.71 / 28.81 (t, C-4',-5'), 29.07 / 31.05 (t, C-2',-7'), 31.54 / 31.70 (t, C-9',-12'), 48.19 (d, C-10'), 128.79 (s, C-1'), 130.27 (s, C-8'), 211.86 (s, C-1). - MS (EI): m/z (%) = 43 (87) [C₂H₃O⁺], 67 (100) [C₅H₇⁺], 81 (68) [C₆H₉⁺], 93 (20) / 107 (15) / 121 (15) / 135 (7) 149 (5) [CₙH₍₂ₙ₋₅₎⁺-Serie], 163 (49) [M⁺-C₂H₃O], 191 (4) [M⁺-CH₃], 206 (23) [M⁺]. - C₁₄H₂₂O (206.33): ber. C 81.50, H 10.75; gef. C 81.49, H 10.57.

Die Verbindungen der Beispiele 2-5 sind in analoger Weise aus 1.2-Bis(methylen)-cyclooctan zugänglich. Von ihnen sind daher nur die Geruchscharakteristik, die spektroskopischen Daten und in einigen Fällen die Elementaranalysen aufgeführt.

### Beispiel 2

### 1-{Bicyclo[6.4.0]dodec-1'(8')-en-10'-yl}methanal (2)

Geruch: Maritim, nach Wassermelonen, Tabak bzw. zigarrenartig und etwas jononig-fruchtig. - IR (Film): ν = 1726 cm⁻¹ (ν HC=O). - ¹H-NMR (CDCl₃): δ = 1.41-1.65 (m, 10H, 3'-H₂-6'-H₂, 11'-H₂), 1.93-2.13 (m, 8H, 2'-,7'-,9'-,12'-H₂), 2.47 (m_{c}, 1H, 10'-H), 9.69 (d, J 1.2 Hz, 1H, CHO). - ¹³C-NMR (CDCl₃): δ = 22.78 (t, C-11'), 26.49 / 26.52 (t, C-3',-6'), 27.99 / 28.52 (t, C-4',-5'), 28.79 / 28.81 (t, C-2',-7'), 31.70 / 31.77 (t, C-9',-12'), 46.81 (d, C-10'), 128.43 (s, C-1'), 130.82 (s, C-8'), 204.82 (d, C-1). - MS (EI): m/z (%) = 29 (33) [CHO], 41 (72) [C₃H₅⁺], 67 (90) [C₅H₇⁺], 91 (98) [C₇H₇⁺], 107 (51) / 121 (34) / 135 (29) / 149 (12) [CₙH₍₂ₙ₋₅₎⁺-Serie], 164 (31) [M⁺-CO], 192 (100) [M⁺].

### Beispiel 3

### 1-{10'-Methylbicyclo[6.4.0]dodec-1'(8')-en-10'-yl}methanal (6)

Geruch: Fruchtig-jononig, ambra- und tabakartig, mit einer Wassermelonen-Note. - IR (Film): ν = 1727 cm⁻¹ (ν HC=O). - ¹H-NMR (CDCl₃): δ = 1.04 (s, 3H, 10'-Me), 1.37-1.54 (m, 10H, 3'-H₂-6'-H₂, 11'-H₂), 1.78 (d, J 16.0 Hz, 1H, 9'-H_{b}), 2.01-2.12 (m, 6H, 2'-,7'-,12'-H₂), 2.29 (d, J 16.0 Hz, 1H, 9'-Hₐ), 9.47 (s, 1H, CHO). - ¹³C-NMR (CDCl₃): δ = 20.68 (q, 10'-Me), 26.42 / 26.48 / 26.61 (t, C-3',-6',-11'), 28.80 / 28.91 (t, C-4',-5'), 29.09 (t, C-12'), 31.63 / 31.86 (t, C-2',-7'), 35.88 (t, C-9'), 45.08 (s, C-10'), 128.17 (s, C-1'), 130.07 (s, C-8'), 206.19 (d, C-1). - MS (EI): m/z (%) = 29 (36) [CHO], 41 (78) [C₃H₅⁺], 67 (65) [C₅H₇⁺], 81 (100) [C₆H₉⁺], 95 (61) [C₇H₁₁⁺], 121 (39) 135 (29) 149 (20) / 163 (28) [CₙH₍₂ₙ₋₅₎⁺-Serie], 177 (70) [M⁺-CHO], 191 (9) [M⁺-CH₃], 206 (31) [M⁺].

### Beispiel 4

### (r-10', c-11')-1-{11'-Methylbicyclo[6.4.0]dodec-1'(8')-en-10'-yl}ethan-1-on (7)

Geruch: Holzig, jononartig, etwas nach Kaffee. - IR (Film): ν = 1710 cm⁻¹ (ν C=O). - ¹H-NMR (CDCl₃): δ = 0.93 (d, J 6.4 Hz, 3H, 11'-Me), 1.35-1.55 (m, 8H, 3'-H₂-6'-H₂), 1.76-2.16 (m, 9H, 2'-,7'-,9'-,12'-H₂, 11'-Hₐₓ), 2.17 (s, 3H, 2-H₃), 2.36 (ddd, J 10.3, 10.3, 5.4 Hz, 1H, 10'-Hₐₓ). - ¹³C-NMR (CDCl₃): δ = 19.48 (q, 11'-Me), 26.44 / 26.25 (t, C-3',-6'), 28.70 / 28.74 (t, C-4',-5'), 29.21 (q, C-2), 30.83 (d, C-11'), 31.30 / 31.42 / 32.47 (t, C-2',-7',-9'), 37.78 (t, C-12'), 55.17 (d, C-10'), 128.26 (s, C-1'), 129.95 (s, C-8'), 213.01 (s, C-1). - MS (EI): m/z (%) = 43 (61) [C₂H₃O⁺], 67 (100) [C₅H₇⁺], 95 (63) [C₇H₁₁⁺], 177 (52) [M⁺-C₂H₃O], 205 (5) [M⁺-CH₃], 220 (19) [M⁺]. - C₁₅H₂₄O (220.35): ber. C 81.76, H 10.98; gef. C 81.46, H 10.95.

### Beispiel 5

### 1-{Bicyclo[6.4.0]dodec-1'(8')-en-10'-yl}propan-1-on (8)

Geruch: Nach β-Jonon, fruchtig-floral, frisch, etwas birnig. - IR (Film): ν = 1711 cm⁻¹ (ν C=O). - ¹H-NMR (CDCl₃): δ = 1.06 (t, J 7.2 Hz, 3H, 3-H₃), 1.35-1.58 (m, 10H, 3'-H₂-6'-H₂, 11'-H₂), 1.99-2.18 (m, 8H, 2'-,7'-,9'-,12'-H₂), 2.48 (dq, J 17.6, 7.3 Hz, 1H, 2-H_{b}), 2.54 (dq, J 17.6, 7.3 Hz, 1H, 2-Hₐ), 2.57 (dddd, J 11.6, 10.4, 5.2, 2.9 Hz, 1H, 10'-H). - ¹³C-NMR (CDCl₃): δ = 7.68 (q, C-3), 25.45 (t, C-11'), 26.45 / 26.47 (t, C-3',-6'), 28.70 / 28.80 (t, C-4',-5'), 29.12 / 31.31 (t, C-2',-7'), 31.53 / 31.69 (t, C-9',-12'), 33.68 (t, C-2), 47.23 (d, C-10'), 128.90 (s, C-1'), 130.17 (s, C-8'), 214.31 (s, C-1). - MS (EI): m/z (%) = 29 (33) [C₂H₅⁺], 57 (40) [C₃H₅O⁺], 67 (100) [C₅H₇⁺], 81 (81) [C₆H₉⁺], 91 (29) [C₇H₇⁺], 107 (13) / 121 (15) 135 (5) [CₙH₍₂ₙ₋₅₎⁺-Serie], 163 (49) [M⁺-C₂H₅-CO], 191 (16) [M⁺-C₂H₅], 220 (18) [M⁺]. - C₁₅H₂₄O (220.35): ber. C 81.76, H 10.98; gef. C 81.80, H 10.90.

### Beispiel 6

### (r-9', c-10')-1-{9',10'-Dimethylbicyclo[6.4.0]dodec-1'(8')-en-10'-yl}-ethan-1-on (3)

### a) Herstellung des Zwischenproduktes 4-Methylenspiro[2.7]decan (V, n=2)

Die Ausgangsverbindung, Spiro[2.7]decan-4-on, wurde nach einer Vorschrift von S. M. Ruder, R. C. Ronald [Tetrahedron Lett. 1984, 25, 5501] hergestellt.

Zu einer Lösung von 19.1 g (170 mmol) Kalium-*tert*-butylat in 350 ml trockenem Tetrahydrofuran wurden unter Rühren und Stickstoff 64.3 g (180 mmol) Methyltriphenylphosphoniumbromid gegeben und zum Rückfluss erhitzt. In die siedende Reaktionsmischung liess man darauf eine Lösung von 22.8 g (150 mmol) Spiro[2.7]decan-4-on in 50 ml trockenem Tetrahydrofuran zutropfen und erhitzte weitere 2 h unter Rückfluss. Nach dem Abkühlen wurde das Reaktionsgemisch auf 1l *tert*-Butylmethylether / Wasser (1:1) gegossen, die organische Phase abgetrennt und die wässrige Phase zweimal mit 500 ml *tert*-Butylmethylether extrahiert. Die organischen Extrakte wurden vereinigt, über Natriumsulfat getrocknet und am Rotationsverdampfer eingeengt. Nach Flashchromatographie (*n*-Pentan, *R*_{f} = 0.97) an Kieselgel erhielt man 17.1 g (76 %) 4-Methylenspiro[2.7]decan (V, n = 2) als farblose Flüssigkeit.

Geruch: frisch minzig, grün-kienig, etwas holzig-fruchtig. - IR (Film): ν 2922 / 2851 / 2997 / 3076 cm⁻¹ (ν C-H), 1444 cm⁻¹ (δ H-C-H), 879 / 1014 cm⁻¹ (δ C=C-H), 1632 cm⁻¹ (ν C=C). - ¹H-NMR (CDCl₃): δ = 0.45 (dd, J 5.8, 3.6 Hz, 2H, 2-H₂), 0.59 (dd, J 5.8, 4.0 Hz, 2H, 1-H₂), 1.45-1.59 (m, 8H, 7-H₂-10-H₂), 1.67-1.73 (m, 2H, 6-H₂), 2.25 (ddd, J 14.6, 6.4, 1.2 Hz, 2H, 5-H₂), 4.71 (dd, J 3.6, 1.2 Hz, 2H, 11-H₂). - ¹³C-NMR (CDCl₃): δ = 14.85 (2t, C-1,-2), 25.00 (s, C-3), 25.24 (t, C-6), 26.38 / 26.41 (t, C-7,-8), 29.53 (t, C-9), 34.36 (t, C-5), 37.09 (t, C-10), 108.88 (t, C-11), 155.76 (s, C-4). - MS (EI): m/z (%) = 67 (85) [C₅H₇⁺], 79 (100) / 93 (61) / 107 (29) / 121 (21) / 135 (13) [CₙH₍₂ₙ₋₅₎⁺-Serie], 150 (3) [M⁺].

### b) Herstellung von (r-9',c-10')-1-{9',10'-Dimethylbicyclo[6.4.0]dodec-1'(8')-en-10'-yl}-ethan-1-on (3)

2.20 g (14.6 mmol) 4-Methylenspiro[2.7]decan wurden in 20 ml trockenem Toluol gelöst und unter Stickstoff mit 550 mg (0.59 mmol, 4 mol%) Tris(triphenylphosphin)rhodium(I)-chorid versetzt. Unter Rühren wurde unter Inertgasatmosphäre 1.80 ml (17.5 mmol) 3-Methyl-3-buten-2-on injiziert und das Reaktionsgemisch darauf 40 h zum Rückfluss erhitzt. Nach dem Abkühlen wurde der Katalysator durch Filtration über Kieselgel abgetrennt, das Filtrat in 400 ml *tert*-Butylmethylether / Wasser (1:1) gegossen und die organische Phase abgetrennt. Die wässrige Phase wurde dreimal mit 200 ml *tert*-Butylmethylether extrahiert und die organischen Extrakte anschliessend vereinigt. Nach Trocknen über Natriumsulfat, Einengen am Rotationsverdampfer und Flashchromatographie (*n*-Pentan/ *tert*-Butylmethylether, 40:1, *R*_{f} = 0.41) an Kieselgel erhielt man 1.62 g (47 %) (*r*-9', *c*-10')-1-{9',10'-Dimethylbicyclo[6.4.0]dodec-1'(8')-en-10'-yl}ethan-1-on (3) als farblosen Feststoff.

Geruch: Intensiv holzig-ambraartig, frisch-trocken weihrauchartig, an Iso E super® aber auch an Vetiveröl erinnernd. - Schmp. 56.3°C. - IR (KBr): ν = 1704 cm⁻¹ (ν C=O). - ¹H-NMR (CDCl₃): δ = 0.83 (d, J 6.8 Hz, 3H, 9'-Me), 1.10 (s, 3H, 10'-Me), 1.42-2.06 (m, 15H, 2'-,7'-H_{b}, 3'-H₂-6'-H₂, 9'-H, 11'-,12'-H₂), 2.14 (s, 2-H₃), 2.23-2.40 (m, 2H, 2'-,7'-Hₐ). - ¹³C-NMR (CDCl₃): δ = 17.06 (q, 9'-Me), 21.04 (q, 10'-Me), 22.73 (t, C-11'), 25.36 (q, C-2), 26.62 / 26.67 (t, C-3',-6'), 26.98 (t, C-4'), 28.75 (t, C-5'), 29.73 (t, C-12'), 30.81 (t, C-7'), 31.68 (t, C-2'), 40.74 (d, C-9'), 49.86 (s, C-10'), 128.85 (s, C-1'), 133.09 (s, C-8'), 214.22 (s, C-1). - MS (EI): m/z (%) = 95 (31) [C₇H₁₁⁺], 191 (100) [M⁺-C₂H₃O], 219 (3) [M⁺-CH₃], 234 (12) [M⁺]. - C₁₆H₂₆O (234.38): ber. C 81.99, H 11.18, O 6.83; gef. C 81.68, H 11.15, O 6.89.

Die Verbindungen der Beispiele 7-10 sind in analoger Weise aus 4-Methylenspiro[2.7]decan zugänglich. Von ihnen sind daher ebenso wie bei den Beispielen 2-5 nur die Geruchsbeschreibung sowie die analytischen Daten angegeben.

### Beispiel 7

### (r-8', c-9')-1-{8',9'-Dimethylbicyclo[5.4.0]undec-1'(7')-en-9'-yl}ethan-1-on (4)

Geruch: Holzig-cedrig, Vetiver, floral, sandelholzartig. - IR (Film): ν = 1703 cm⁻¹ (ν C=O) . - ¹H-NMR (CDCl₃): δ = 0.77 (d, J 6.8 Hz, 3H, 8'-Me), 1.06 (s, 3H, 9'-Me), 1.34-1.44 (m, 5H, 4'-H₂, 8'-H, 10'-H₂), 1.68-1.75 (m, 4H, 3'-,5'-H₂), 1.93-2-08 (m, 6H, 2'-,6'-,11'-H₂), 2.11 (s, 3H, 2-H₃). - ¹³C-NMR (CDCl₃): δ = 16.32 (q, 8'-Me), 20.59 (q, 9'-Me), 22.97 (t, C-10'), 25.39 (q, C-2), 26.26 / 26.82 (t, C-3',-5'), 28.83 (t, C-4'), 32.76 (t, C-11'), 34.40 / 34.58 (t, C-2',-6'), 43.34 (d, C-8'), 49.54 (s, C-9'), 132.12 (s, C-1'), 135.45 (s, C-7'), 214.14 (s, C-1). - MS (EI): m/z = 43 (52) [C₂H₃O⁺], 95 (100) [C₇H₁₁⁺], 107 (40) / 121 (32) / 149 (4) [CₙH₍₂ₙ₋₅)⁺-Serie], 177 (81) [M⁺-C₂H₃O], 220 (7) [M⁺].

### Beispiel 8

### (r-9', c-10')-1-{9'-Methylbicyclo[6.4.0]dodec-1'(8')-en-10'-yl}ethan-1-on (5)

Geruch: Holzig, nach Vetiver und β-Jonon, mit einer leichten Ambra-Note. -IR (Film): ν = 1709 cm⁻¹ (ν C=O). - ¹H-NMR (CDCl₃): δ = 0.84 (J 6.8 Hz, 3H, 9'-Me), 1.39-1.61 (m, 8H, 3'-H₂-6'-H₂), 1.72 (m_{c}, 2H, 11'-H₂), 1.93-2.08 (m, 4H, 2'-H₂, 7'-,12'-H_{b}), 2.16 (s, 3H, 2-H₃), 2.19-2.31 (m, 2H, 7'-,12'-Hₐ), 2.52 (m_{c}, 1H, 9'-H), 2.56 (m_{c}, 1H, 10'-H). - ¹³C-NMR (CDCl₃): δ = 14.65 (q, 9'-Me), 18.07 (t, C-11'), 26.38 / 26.90 (t, C-3',-6'), 28.39 (q, C-2), 28.46 / 29.20 (t, C-4',-5'), 30.21 / 30.83 (t, C-2',-7'), 31.40 (C-12'), 34.50 (d, C-9'), 52.63 (d, C-10'), 130.53 / 134.38 (s, C-1',-8'), 211.46 (s, C-1). - MS (EI): m/z (%) = 43 (65) [C₂H₃O⁺], 81 (100) [C₆H₉⁺], 95 (50) [C₇H₁₁⁺], 177 (38) [M⁺-C₂H₃O], 205 (3) [M⁺-CH₃], 220 (14) [M⁺]. - C₁₅H₂₄O (220.35): ber. C 81.76, H 10.98; gef. C 81.60, H 10.87.

### Beispiel 9

### (r-9',c-10',t-11')-1-{9',11'-Dimethylbicyclo[6.4.0]dodec-1'(8')-en-10'yl}ethan-1-on (9)

Geruch: Holzig, balsamisch, fruchtig, relativ schwach und ein wenig nach β-Jonon. - IR (Film): ν = 1710 cm⁻¹ (ν C=O). - ¹H-NMR (CDCl₃): δ = 0.84 (d, J 7.2 Hz, 3H, 11'-Me), 0.91 (d, J 6.0 Hz, 3H, 9'-Me), 1.42-1.55 (m, 8H, 3'-H₂-6'-H₂), 1.67 (dd, J 16.8, 10.4 Hz, 1H, 12-H_{b}), 1.89-2.27 (m, 5H, 2'-H₂,7'-H₂,12'-Hₐ), 2.09 (m_{c},1H, 11-Hₐₓ), 2.15 (s, 3H, 2-H₃), 2.37 (dq, J 6.0, 4.8 Hz, 1H, 9-H_{eq}), 2.45 (dd, J 11.1, 4.8 Hz, 1H, 10-Hₐₓ). - ¹³C-NMR (CDCl₃): δ = 15.30 (q, 9'-Me), 19.93 (q, 11'-Me), 23.85 (d, C-11'), 26.37 / 26.90 (t, C-3',-6'), 28.41 (t, C-5'), 30.14 (t, C-4'), 30.52 (q, C-2), 30.65 (t, C-7'), 31.23 (t, C-2'), 35.26 (d, C-9'), 38.46 (t, C-12'), 59.06 (d, C-10'), 129.88 (s, C-1'), 134.10 (s, C-8'), 211.05 (s, C-1) - MS (EI): m/z (%) = 43 (68) [C₂H₃O⁺], 95 (100) [C₇H₁₁⁺], 109 (45) [C₈H₁₃⁺], 191.1797 (38) [M⁺-C₂H₃O], 219 (5) [M⁺-CH₃], 234 (11) [M⁺].

### Beispiel 10

### 1-{8'-Methylbicyclo[5.4.0]undec-1'(7')-en-9'-yl}ethan-1-on (10)

Geruch: Jononig, grün-floral, holzig, cremig. - IR (Film): ν = 1709 cm⁻¹ (ν C=O), 1353 / 1376 cm⁻¹ (δ CH₃). - ¹H-NMR (CDCl₃): δ = 0.80 (d, 7.1 Hz, 3H, 8'-Me), 1.34-1.76 (m, 8H, 3'-H₂-5'-H₂,10'-H₂), 1.99-2.15 (m, 6H, 2'-,6'-,11'-H₂), 2.16 (s, 3H, 2-H₃), 2.45 (quint, J 6.2 Hz, 1H, 8'-H), 2.58 (ddd, J 12.1, 6.2, 3.2 Hz, 1H, 9'-H). - ¹³C-NMR (CDCl₃): δ = 14.12 (q, 8'-Me), 18.26 (t, C-10'), 26.23 / 27.25 (t, C-3',-5'), 28.44 (q, C-2), 31.59 / 32.71 (t, C-2',-6'), 34.12 / 34.68 (t, C-4',-11'), 36.82 (d, C-8'), 52.32 (d, C-9'), 134.00/ 137.29 (s, C-1',-7'), 211.53 (s, C-1). - MS (EI): m/z (%) = 28 (38) [CO⁺], 43 (68) [C₂H₃O⁺], 81 (100) [C₆H₉⁺], 91 (34) [C₇H₇⁺], 105 (24) [C₇H₇O⁺], 121 (13) [C₉H₁₃⁺], 163 (38) [M⁺-C₂H₃O], 191 (2) [M⁺-CH₃], 206 (7) [M⁺].

### Beispiel 11

### (r-8',c-10')-1-{Bicyclo[6.4.0]dodec-1'(12')-en-10'-yl}ethan-1-on (11)

Das benötigte Dien, 1-Vinylcyclooct-1-en, wurde nach W. Herz und R.-R. Juo [*J. Org. Chem.* **1985**, *50*, 618] synthetisiert.

3.60 ml (44.1 mmol) But-3-en-2-on wurden unter Rühren zu einer Lösung von 3.50 g (36.8 mmol) 1-Vinylcyclooct-1-en in 40 ml trockenem Toluol gegeben. Nach Abkühlen auf 0 °C fügte man unter Stickstoff 0.41 g (3.05 mmol, 8 mol%) Aluminiumtrichlorid hinzu. Nach 15 min. Rühren bei 0 °C entfernte man die Kühlung und ließ auf Raumtemperatur erwärmen. Bei dieser Temperatur wurde dann 6 Tage unter Stickstoff rühren gelassen und die Reaktionsmischung darauf auf 200 ml Wasser gegossen. Man extrahierte dreimal mit je 200 ml *tert*-Butylmethylether, trocknete die vereinigten oganischen Extrakte über Magnesiumsulfat und entfernte das Lösungsmittel am Rotationsverdampfer. Flashchromatographie (*n*-Pentan / *tert*-Butylmethylether, 50:1, *R*_{f} = 0.46) an Kieselgel lieferte 1.79 g (24 %) (*r*-8',*c*-10')-1-{Bicyclo[6.4.0]dodec-1'(12')-en-10'-yl)ethan-1-on (**11**) als Hauptkomponente im Gemisch mit dem regioisomeren (*r*-8',*c*-9')-1-{Bicyclo[6.4.0]dodec-1'(12')-en-9'yl}ethan-1-on. Obwohl beide Isomere ähnlich riechen, wird der Geruch der Mischung von dem intensiveren (*r*-8',*c*-10')-1-{Bicyclo[6.4.0]dodec-1'(12')-en-10'yl}ethan-1-on (**11**) bestimmt.

Geruch: holzig-fruchtig, jononig, Raldein, Vertenex, cedrig. - IR (Film): ν = 1709 cm⁻¹ (ν OC=O). - ¹H-NMR (CDCl₃): δ = 1.33-1.72 (m, 11H, 2'-H, 4'-H₂-7'-H₂, 9'-H₂), 1.92-2.16 (m, 4H, 3'-,11'-H₂), 2.18 (s, 3H, 2-H₃), 2.19-2.39 (m, 2H, 2'-,8'-H), 2.66 (m_{c}, 1H, 10'-H), 5.42 (t, *J* 3.1 Hz, 1H, 12'-H). - ¹³C-NMR-Daten des Hauptisomers (CDCl₃): δ = 24.64 / 25.17 (t, C-3',-6'), 25.93 (t, C-11'), 27.10 / 27.79 (t, C-4',-5'), 28.00 (q, C-2), 29.59 (t, C-7'), 32.62 (t, C-2'), 36.80 (t, C-9'), 38.17 (d, C-8'), 48.37 (d, C-10'), 120.24 (d, C-12'), 143.46 (s, C-1'), 211.98 (s, C-1). - MS (EI): *m*/*z* (%) = 43 (100) [C₂H₃O⁺], 55 (29) [C₄H₇⁺], 67 (62) [C₅H₇⁺], 81 (67) [C₆H₉⁺], 91 (38) [C₇H₇⁺], 107 (13) / 121 (15) [CₙH₍₂ₙ₋₅₎⁺-Serie], 163 (30) [M⁺-C₂H₃O], 191 (3) [M⁺-CH₃], 206 (6) [M⁺]. - C₁₄H₂₂O (206.33): ber. C 81.50, H 10.75; gef. C 81.38, H 10.56.

Die Verbindungen der folgenden Beispiele sind in analoger Weise aus 1-Vinylcyclooct-1-en zugänglich. Da sie ebenfalls im Gemisch mit den regio- und diastereoisomerern Diels-Alder Addukten auftreten, wurden die Verbindungen zur eindeutigen Charakterisierung teilweise mikropräparativ aufgereinigt und analysiert.

### Beispiel 12

### (r-8',c-10')-1-{Bicyclo[6.4.0]dodec-1'(12')-en-10'-yl}methanal (12)

Geruch: maritim, nach Wassermelone, Tabak und Holz, etwas jononig-fruchtig und leicht ozonig. - IR (Film): ν = 1724 cm⁻¹ (ν CHO). - ¹H-NMR (CDCl₃): δ = 1.29-1.92 (m, 10H, 3'-H₂-6'-H₂,7'-,9'-H_{b}), 1.87 (m_{c}, 1H, 9'-Hₐ), 1.99 (ddd, *J* 13.8, 13.6, 3.1 Hz, 1H, 2'-H_{b}), 2.10 (m_{c}, 1H, 7'-Hₐ), 2.07-2.26 (m, 2H, 11'-H₂), 2.27 (m_{c}, 1H, 2'-Hₐ), 2.36 (m_{c}, 1H, 8'-H), 2.55 (m_{c}, 1H, 10'-H), 5.42 (t, *J* 2.5 Hz, 1H, 12'-H), 9.70 (d, *J* 1.6 Hz, 1H, 1-H). - ¹³C-NMR-Daten des Hauptisomers (C₆D₆): δ = 24.68 / 25.54 / 25.38 / 26.28 (t, C-3'-C-6'), 25.45 (t, C-11'), 27.44 (t, C-7'), 28.33 (t, C-9'), 32.81 (t, C-2'), 38.12 (d, C-8'), 47.58 (d, C-10'), 120.43 (d, C-12'), 143.64 (s, C-1'), 202.72 (d, C-1). - MS (EI): *m*/*z* (%) = 29 (10) [CHO⁺], 41 (32) [C₃H₅⁺], 67 (26) [C₅H₇⁺], 91 (100) [C₇H₇⁺], 79 (45) / 93 (34) 107 (41) / 121 (33) / 135 (14) / 149 (6) [CₙH(₂ₙ₋₅)⁺-Serie], 192 (63) [M⁺].

### Beispiel 13

### (r-8',c-10')-1-{10'-Methylbicyclo[6.4.0]dodec-1'(12')-en-10'-yl}ethan-1-on (13)

Geruch: holzig-jononig, cremig, warm, süss, ambriert, nach Iso E super und Vertofix. Im Vergleich mit den ebenfalls durch präparative GC erhaltenen Proben der Regio- und Diastereoisomere besitzt die Verbindung 13 den interessantesten Geruch und den niedrigsten Schwellenwert. - IR (Benzol): ν = 1705 cm⁻¹ (ν OC=O). - ¹H-NMR (CDCl₃): δ = 1.14 (s, 3H, 10'-Me), 1.34-1.73 (m, 8H, 3'-H₂-6'-H₂), 1.42 (m_{c}, 1H, 7'-H_{b}), 1.52 (dd, *J* 13.0, 4.9 Hz, 1H, 9'-H_{b}), 1.66 (dd, *J* 13.0, 12.2 Hz, 1H, 9'-Hₐ), 1.83 (m_{c}, 1H, 7'-Hₐ), 1.89 (dd, 1H, *J* 15.0, 3.3 Hz, 1H, 11'-H_{b}), 1.99 (ddd, *J* 13.4, 12.5, 3.5 Hz, 1H, 2'-H_{b}), 2.17 (s, 3H, 2-Me), 2.26 (m_{c}, 1H, 2'-Hₐ), 2.29 (m_{c}, 1H, 8'-H), 2.31 (dd, 1H, *J* 15.0, 3.3 Hz, 1H, 11'-Hₐ), 5.37 (t, *J* 3.3 Hz, 1H, 12'-H). - ¹³C-NMR (CDCl₃): δ = 20.26 (q, 10'-Me), 24.77 (q, C-2), 24.78/ / 25.23 (t, C-3',-6'), 26.02 / 27.07 (t, C-4',-5'), 32.56 (t, C-2'), 32.78 (t, C-7'), 33.60 (t, C-11'), 34.47 (d, C-8'), 34.72 (t, C-9'), 46.65 (s, C-10'), 119.42 (d, C-12'), 142.04 (s, C-1'), 214.48 (s, C-1). - MS (EI): *m*/*z* (%) = 43 (75) [C₂H₃O⁺], 55 (37) [C₄H₇⁺], 59 (58) [C₃H₇O⁺], 67 (29) [C₅H₇⁺], 81 (100) [C₆H₉⁺], 95 (54) [C₇H₁₁⁺], 107 (14) / 121 (19) 135 (8) / 149 (3) (CₙH₍₂ₙ₋₅₎⁺-Serie], 177 (30) [M⁺-C₂H₃O], 205 (29) [M⁺-CH₃], 220 (16) [M⁺].

### Beispiel 14

### (r-7',c-9')-1-{Bicyclo[5.4.0]undec-1'(11')-en-9'-yl}ethan-1-on (14)

Geruch: süss, jononig-fruchtig, holzig-frisch, etwas nach Himbeere. - IR (Film): ν = 1709 cm⁻¹ (ν OC=O). - ¹H-NMR (CDCl₃): δ = 1.36-1.88 (m, 10H, 3'-H₂-6'-H₂,8'-H₂), 2.08-2.22 (m, 4H, 2'-,10'-H₂), 2.17 (s, 3H, 2-H₃), 2.32 (m_{c}, 1H, 7'-H), 2.63 (dddd, *J* 12.0, 11.6, 5.7, 2.9 Hz, 1H, 9'-H), 5.41 (m_{c}, 1H, 11'-H). -¹³C-NMR (CDCl₃): δ = 26.30 / 27.32 (t, C-3',-5'), 27.86 (q, C-2), 29.85 (t, C-4'), 31.13 (t, C-6'), 33.35 / 33.39 (t, C-8',-10'), 35.70 (t, C-2'), 39.21 (d, C-7'), 48.10 (d, C-9'), 120.34 (d, C-11'), 143.06 (s, C-1'), 211.68 (s, C-1). - MS (EI): *m*/*z* (%) = 43 (24) [C₂H₃O⁺], 55 (6) [C₄H₇⁺], 67 (44) [C₅H₇⁺], 91 (44) [C₇H₇⁺], 79 (18) / 93 (16) / 107 (12) / 121 (8) [CₙH₍₂ₙ₋₅₎⁺-Serie], 149 (100) [M⁺-C₂H₃O], 159 (3) [M⁺-CH₃-H₂O], 177 (14) [M⁺-CH₃], 192 (34) [M⁺].

### Beispiel 15

### Damen-Extrait-Parfüm mit Verbindung 3

| | Verbindung / Bestandteil | Gewichtsanteil in‰ |
|---|---|---|
| 1. | AMBRAROME | 15 |
| 2. | CEDERNHOLZÖL VIRGINIA | 50 |
| 3. | CITRONELLOL EXTRA | 30 |
| 4. | CYCLOPENTADECANOLID (THIBETOLID) | 50 |
| 5. | 4-(1-ETHOXYETHENYL)-3,3,5,5-TETRAMETHYLCYCLOHEXANON (KEPHALIS) | 40 |
| 6. | 2-ETHYL-6,6-DIMETHYLCYCLOHEX-2-ENCARBONSÄUREETHYLESTER (GIVESCONE) | 15 |
| 7. | ETHYL LINALOOL | 50 |
| 8. | EUGENOL REIN | 30 |
| 9. | 3-(4-METHOXYPHENYL)-2-METHYLPROPANAL | 60 |
| 10. | 1,2,3,5,6,7-HEXAHYDRO-1,1,2,3,3-PENTAMETHYL-4*H*-INDENON (CASHMERAN) | 15 |
| 11. | INDOL 10% DPG | 2 |
| 12. | ISOAMBRETTOLID (AMBRETTOLID) | 15 |
| 13. | KÜMMELÖL RÖMISCH (CUMINÖL) 10% DPG | 4 |
| 14. | LINALYLACETAT SYNTHETISCH | 30 |
| 15. | MANDARINENÖL | 20 |
| 16. | METHYL ANTHRANILAT 10% DPG | 3 |
| 17. | METHYLJONON (ISORALDEINE 95) | 120 |
| 18. | NECTARYL | 10 |
| 19. | 3-METHYL-5-(2,2,3-TRIMETHYLCYCOPENT-3-EN-1-YL)PENTAN-2-OL (SANDALORE) | 90 |
| 20. | OLIBANUMÖL REIN | 5 |
| 21. | PATCHOULIÖL REIN | 40 |
| 22. | PHENYLETHYLALKOHOL EXTRA | 32 |
| 23. | ROSENÖL | 2 |
| 24. | VANILLIN | 4 |
| **25.** | **VERBINDUNG 3** | **250** |
| 26. | WACHOLDERBEERÖL | 10 |
| 27. | YLANG-YLANG-ÖL EXTRA | 5 |
| 28. | ZIMTALDEHYD | 3 |
| | | 1000 |

Die Verbindung 3 bildet zusammen mit Methyljonon, Patchouli, Sandalore und Thibetoid/Ambrettolid den Hauptakkord dieses modernen, monolithischen feminin-holzigen Chypre-Parfüms. Trotz der hohen Dosis von 25% bleibt die Komposition harmonisch und transparent, und lebt von der cedrigambraartigen, angenehmen Wärme der Verbindung 3. Diese entfaltet sich bereits erfrischend in dem aromatischen Angeruch und entwickelt sich in einem würzig-blumigem Bukett, das sie langanhaltend fixiert. In Geruchsintensität, Diffusivität und Haftfestigkeit ist die Verbindung 3 bei gleicher Dosierung den Riechstoffen Iso E super und Vertofix in dieser Komposition deutlich überlegen.

### Beispiel 16

### Parfümöl für Weichspüler mit Verbindung 3

| | Verbindung / Bestandteil | Gewichtsanteil in ‰ |
|---|---|---|
| 1. | AMYLSALICYLAT | 50 |
| 2. | BENZYLAKOHOL EXTRA | 100 |
| 3. | CITRONELLOL EXTRA | 50 |
| 4. | CUMARIN REIN KRISTALLIN | 20 |
| 5. | CYCOPENTADECANOLID (THIBETOLID) | 30 |
| 6. | 2-METHYL-3-[4-(1,1-DIMETHYL)ETHYL]PHENYLPROPANAL (LILIAL) | 250 |
| 7. | DYNASCON 10 | 1 |
| 8. | 3-(4-ETHYLPHENYL)-2,2-DIMETHYLPROPANAL (FLORALOZONE) | 4 |
| 9. | 2-ETHYL-4-(2,2,3-TRIMETHYLCYCLOPENT-3-EN-1-YL)BUT-2-EN-1-OL (RADJANOL) | 10 |
| 10. | ALPHA-HEXYLZIMTALDEHYD | 150 |
| 11. | LINALOOL SYNTHETISCH | 100 |
| 12. | PARA-METHYLACETOPHENON | 5 |
| 13. | METHYLJONON (ISORALDEINE 70) | 100 |
| 14. | PHENYLETHYLACETAT | 30 |
| 15. | TERPINEOL REIN | 50 |
| **16.** | **VERBINDUNG 3** | **50** |
| | | 1000 |

Die Verbindung 3 verleiht der Komposition eine angenehme frisch-trockene Note mit einer weichen, weihrauchartigen Wärme, die den weichpflegenden Produktcharakter unterstreicht. Sie rundet die Komposition ab, besitzt eine sehr gute Haftfähigkeit auf der Wäsche und wirkt fixierend auf die übrigen Riechstoffe.

### Beispiel 17

### Floral-maritime Parfüm-Komposition mit Verbindung 2

| | Verbindung / Bestandteil | Gewichtsanteil in ‰ |
|---|---|---|
| 1. | BERGAMOTTÖL | 100 |
| 2. | CITRONENÖL ITALIENISCH REIN | 20 |
| 3. | 3-CYCLOHEXYLPROPIONSÄUREALLYLESTER 10% DPG | 5 |
| 4. | ALPHA-DAMASCON 10% DPG | 3 |
| 5. | 2,6-DIMETHYLHEPTAN-2-OL | 1 |
| 6. | 1,1-DIMETHYL-3-PHENYLPROPANOL | 3 |
| 7. | DIPROPYLENGLYKOL (DPG) | 90 |
| 8. | ETHYL LINALOOL | 85 |
| 9. | GERANIOL REIN | 1 |
| 10. | 1,3,4,6,7,8-HEXAHYDRO-4,6,6,7,8,8-HEXAMETHYL-CYCLOPENTA[G]-2-BENZOPYRAN (GALAXOLIDE) 50 BB | 140 |
| 11. | BETA-JONON SYNTHETISCH | 2 |
| 12. | LINALOOL SYNTHETISCH | 35 |
| 13. | LINALYL ACETAT SYNTHETISCH | 15 |
| 14. | 7-METHYL-2*H*-BENZO-1,5-DIOXEPIN-3(4H)-ONE (CALONE 1951) | 6 |
| 15. | 2-METHYL-3-[4-(1,1-DIMETHYL)ETHYL]-PHENYLPROPANAL (LILIAL) | 120 |
| 16. | METHYLDIHYDROJASMONAT (CEPIONAT) | 320 |
| 17. | NONA-2(*E*),6(*Z*)-DIENAL 10%/TEC 10% DPG | 5 |
| 18. | PHENYLACETALDEHYD (HYAZINTH-ALDEHYD) 85%/PEA 10% DPG | 5 |
| 19. | ALPHA-TERPINEOL (LINDENOL) | 2 |
| 20. | GAMMA-UNDECALACTON (PEACH PURE) | 2 |
| **21.** | **VERBINDUNG 2** | **40** |
| | | 1000 |

Die Verbindung 2 prägt die floral-maritime Note dieser Parfüm-Komposition, verleiht ihr aber auch fruchtig-jononige Aspekte und eine angenehme tabakartige Note. Sie fixiert und harmonisiert die Kreation durch ihre floralen, maritimen und holzigen Facetten und bringt Natürlichkeit und Transparenz in das moderne parfümistische Thema "wässrig-blumig".

### Beispiel 18

### Florale Hesperiden-Note mit Verbindung 2

| | Verbindung / Bestandteil | Gewichtsanteil in ‰ |
|---|---|---|
| 1. | BEIFUSSÖL 10% DPG | 6 |
| 2. | BERGAMOTTÖL | 70 |
| 3. | CARDAMOMENÖL CEYLON | 15 |
| 4. | CEDERNHOLZÖL BRAUN VIRGINIA | 150 |
| 5. | CITRONELLOL EXTRA | 20 |
| 6. | CORIANDERÖL | 2 |
| 7. | CUMARIN REIN KRISTALLIN | 2 |
| 8. | CUMINÖL (RÖMISCHES KÜMMELÖL) 10% DPG | 5 |
| 9. | CYPRESSENÖL | 3 |
| 10. | 2,4-DIHYDROXY-3,6-DIMETHYLBENZOESÄURE-METHYLESTER (EVERNYL) 10% DPG | 5 |
| 11. | DIPROPYLENGLYKOL (DPG) | 109 |
| 12. | ETHYL LINALOOL | 45 |
| 13. | (3Z)-HEX-3-EN-1-OL 10% DPG | 5 |
| 14. | ALPHA-HEXYLZIMTALDEHYDE | 17 |
| 15. | BETA-JONON SYNTHETISCH | 30 |
| 16. | MACISÖL (MUSKATNUSSÖL) USP | 10 |
| 17. | MANDARINENÖL GELB ITALIENISCH | 10 |
| 18. | METHYLDIHYDROJASMONAT (CEPIONAT) | 120 |
| 19. | MUSCON 10% DPG | 6 |
| 20. | ORANGENÖL BITTER | 10 |
| 21. | 2-PHENYLETHAN-1-OL WEISS | 10 |
| **22.** | **VERBINDUNG 2** | **350** |
| | | 1000 |

Die Verbindung 2 verstärkt die Intensität und den Charakter des Parfüms, hebt die frisch-hesperidenartigen Facetten hervor und schwächt die trockenholzigen Elemente ab.

## Patentansprüche

1. Verbindungen der Formel I 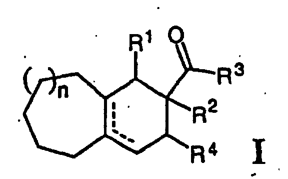
worin n = 1, 2
und R¹, R⁴ = H oder CH₃
und R², R³ = H, CH₃ oder CH₂CH₃
darstellen, mit Ausnahme der Verbindung der Formel I, worin
n = 1 ;
R¹ = R² = R⁴ = H, und
R³ = CH₂CH₃ ist.

2. Verbindungen nach Anspruch 1 gemäs der Formel I' 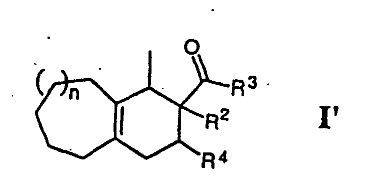
worin n = 1, 2
und R⁴ = H oder CH₃
und R², R³ = H, CH₃ oder CH₂CH₃
darstellen.

3. Verbindung nach Anspruch 1 ausgewählt aus der Gruppe bestehend aus 1-(Bicyclo[6.4.0]dodec-1'(8')-en-10'-yl}ethan-1-on (**1**), 1-(Bicyclo[6.4.0]dodec-1'(8')-en-10'-yl}methanal (**2**), 1-{9',10'-Dimethylbicyclo[6.4.0]dodec-1'(8')-en-10'-yl}ethan-1-on (**3**), 1-{8',9'-Dimethylbicyclo[5.4.0]undec-1'(7')-en-9'-yl}-ethan-1-on (**4**), 1-{9'-Methylbicyclo[6.4.0]dodec-1'(8')-en-10'-yl}ethan-1-on (**5**), 1-{10'-Methylbicyclo[6.4.0]dodec-1'(8')-en-10'-yl}methanal (**6**), 1-{11'-Methyibicyclo[6.4.0]dodec-1'(8')-en-10'-yl}ethan-1-on (**7**), 1-{Bicyclo[6.4.0]dodec-1'(8')-en-10'-yl}propan-1-on (**8**), 1-{9',11'-Dimethylbicyclo[6.4.0]dodec-1'(8')-en-10'-yl}ethan-1-on (**9**), 1-{8'-Methylbicyclo[5.4.0]undec-1'(7')-en-9'-yl}ethan-1-on (**10**), 1-{Bicyclo[6.4.0]dodec-1'(12')-en-10'-yl}ethan-1-on (**11**), 1-{Bicyclo[6.4.0]-dodec-1'(12')-en-10'-yl}methanal (**12**), 1-{10'-Methylbicyclo[6.4.0]dodec-1'(12')-en-10'-yl}-ethan-1-on (**13**) und 1-{Bicyclo[5.4.0]undec-1'(11')-en-9'-yl}ethan-1-on (**14**).

4. Verbindung nach Anspruch 2 ausgewählt aus der Gruppe bestehend aus 1-{9',10'-Dimethylbicyclo[6.4.0]dodec-1'(8')-en-10'-yl}-ethan-1-on (**3**), 1-{8',9'-Dimethylbicyclo[5.4.0]under-1'(7')-en-9'-yl}-ethan-1-on (**4**), 1-{9'-Methylbicyclo[6.4.0]dodec-1'(8')-en-10'-yl}ethan-1-on (**5** ) und 1-{8'-Methylicyclo[5.4.0]undec-1'(7')-en-9'-yl}ethan-1-on (**10**).

5. Verbindung nach Anspruch 3 ausgewählt aus der Gruppe bestehend aus 1-{Bicyclo[6.4.0]dodec-1'(8')-en-10'-yl}methanal (**2**) und 1-{10'-Methylbicyclo[6.4.0]dodec-1'(8')-en-10'-yl}methanal (**6**).

6. Verbindung nach Anspruch 3 ausgewählt aus der Gruppe bestehend aus 1-{Bicyclo[6.4.0]dodec-1'(8')-en-10'-yl}methanal (**2**) und 1-{9',10'-Dimethylbiyclo[6.4.0]dodec-1'(8')-en-10'-yl}ethan-1-on (**3**).

7. Verbindung nach Anspruch 3 ausgewählt aus der Gruppe 1-{Bicyclo-6.4.0]dodec-1'(12')-en-10'-yl}methan-1-on (**11**), 1-{Bicyclo[6.4.0]dodec-1'(12')-en-10'-yl}methanal (**12**), 1-{10'-Methylbicyclo[6.4.0]dodec-1'(12')-en-10'-yl}ethan-1-on (**13**) und 1-{Bicyclo[5.4.0]undec-1'(11')-en-9'-yl}ethan-1-on (**14**) im Gemisch mit ihren entsprechenden regioisomeren Diels-Alder-Addukten.

8. Verwendung einer Verbindung nach einem der Ansprüche 1 bis als Riechstoff.

9. Riechstoffkomposition, **dadurch gekennzeichnet, dass** sie mindestens eine Verbindung aus einem der Ansprüche 1-7 enthält.

10. Verfahren zur Herstellung der R¹⁻methylsubstituierten Verbindungen der Formel I' gemäss Anspruch 2, **dadurch gekennzeichnet, dass** eine spirocyclische Vinylcyclopropanverbindung mit einem Dienophil in Gegenwart eines Katalysators regioselektiv oder Z- diastereoselektiv zu den Zielverbingungen der Formel Γ umgesetzt wird.

11. Verfahren nach Anspruch 10, **dadurch gekennzeichnet, dass** die Vinylcyclopropanverbindung 4-Methylenspiro[2.6]nonan ist.

12. Verfahren nach Anspruch 10, **dadurch gekennzeichnet, dass** die Vinylcyclopropanverbindung 4-Methylenepiro[2.7]decan ist.

13. Verfahren nach einem der Ansprüche 10 bis 12, **dadurch gekennzeichnet, dass** das Dienophil 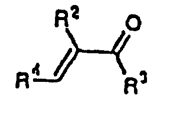 ist.

14. Verfahren nach einem der Ansprüche 10 bis 13, **dadurch gekennzeichnet, dass** der Katalysator (Ph₃P)₃RhCl ist.

15. 4-Methylenspiro[2.6]nonan.

16. Verwendung der Verbindung nach Anspruch 15 oder von 4-methylenspiro[2.7]decan als Riechstoff.

## Claims

1. Compounds of the formula I 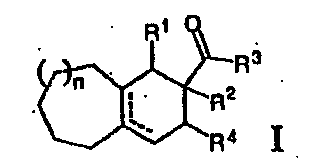
wherein n = 1,2
and R¹, R⁴ = H or CH₃
and R², R³ = H, CH₃ or CH₂CH₃,
with the exception of the compound of the formula I wherein
n = 1
R¹ = R² = R⁴ = H, and
R³ = CH₂CH₃

2. Compounds according to claim 1 of the formula I' 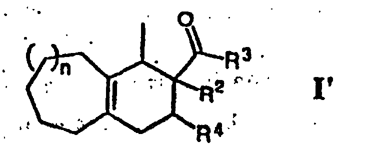
wherein n = 1,2
and R⁴ = H or CH₃
and R², R³ = H, CH₃ or CH₂CH₃

3. Compound according to claim 1 selected from the group consisting of
1-(bicyclo[6.4.0]dodec-1'(8')-en-10'-yl)ethan-1-one (**1**), 1-(bicyclo[6.4.0]dodec-1'(8')-en-10'-yl)methanal (**2**), 1(9',10'-dimethylbicyclo[6.4.0]dodec-1'(8')-en-10'-yl)ethan-1-one (**3**), 1-(8',9'-dimethylbicyclo[5.4.0]undec-1'(7')-en-9'-yl)ethan-1-one (**4**), 1-(9'-methylbicyclo[6.4.0]-dodec-1'(8')-en-10'-yl) ethan-1-one (**5**), 1-(10'-methylbicyclo[6.4.0] dodec-1'(8')-en-10'-yl)-methanal (**6**), 1-(11'-methylbicyclo[6.4.0]dodec-1'(8')-en-10'-yl)ethan-1-one (**7**), 1-(bicyclo[6.4.0]dodec-1'(8')-en-10'-yl)propan-1-one (**8**), 1- (9',11'-dimethylbicyclo[6.4.0]dodec-1'(8') -en-10'-yl)ethan-1-one (**9**), 1-(8'-methylbicyclo[5.4.0]undec-1'(7')-en-9'-yl)ethan-1-one (**10**), 1-(bicyclo[6.4.0]dodec-1'(12') -en-10'-yl) ethan-1-one (**11**), 1-(bicyclo[6.4.0]dodec-1'(12')-en-10'-yl)methanal (**12**), 1-(10'-methylbicyclo[6.4.0]-dodec-1'(12')-en-10'-yl)-ethan-1-one (**13**) and 1-(bicyclo[5.4.0]undec-1'(11')-en-9'-yl)ethan-1-one (**14**).

4. Compound according to claim 2 selected from the group consisting of 1-(9',10'-dimethylbicyclo[6.4.0]dodec-1'(8')-en-10'-yl)ethan-1-one (**3**), 1-(8',9'-dimethylbicyclo[5.4.0]-undec-1'(7')-en-9'-yl)ethan-1-one (**4**), 1-(9'-methylbicyclo[6.4.0]-dodec-1'(8')-en-10'-yl)ethan-1-one (**5**) and 1-(8'-methylbicyclo[5.4.0]undec-1'(7')-en-9'-yl)ethan-1-one (**10**).

5. Compound according to claim 3 selected from the group consisting of 1-(bicyclo[6.4.0]dodec-1'(8')-en-10'-yl)methanal (**2**) and 1- (10'-methylbicyclo[6.4.0]-dodec-1'(8')-en-10'-yl)methanal (**6**).

6. Compound according to claim 3 selected from the group consisting of 1-(bicyclo[6.4.0]dodec-1'(8')-en-10'-yl)methanal (**2**) and 1-(9',10'-dimethylbicyclo[6.4.0]dodec-1'(8')-en-10'-yl)ethan-1-one (**3**).

7. Compound according to claim 3 selected from the group consisting of 1-(bicyclo[6.4.0]dodec-1'(12')-en-10'-yl)ethan-1-one (**11**), 1-(bicyclo[6.4.0]dodec-1'(12')-en-10'-yl)methanal (**12**), 1-(10'-methylbicyclo[6.4.0]dodec-1'(12')-en-10'-yl)ethan-1-one (**13**) and 1-(bicyclo-[5.4.0]-undec-1'(11')-en-9'-yl)ethan-1-one (**14**) in a mixture with their corresponding regioisomeric Diels-Alder adducts.

8. Use of a compound according to one of claims 1 to 7 as an odoriferous substance.

9. Odoriferous substance composition **characterised in that** it contains at least one compound from one of claims 1 to 7.

10. Process for the preparation of the R¹-methyl-substituted compounds of the formula I' according to claim 2, **characterised in that** a spirocyclic vinylcyclopropane compound is reacted regioselectively or Z-diastereo-selectively to the target compounds of the formula I' with a dienophile in the presence of a catalyst.

11. Process according to claim 10, **characterised in that** the vinylcyclopropane compound is 4-methylenespiro[2.6]nonane.

12. Process according to claim 10, **characterised in that** the vinylcyclopropane compound is 4-methylenespiro[2.7]decane.

13. Process according to one of claims 10 to 12 **characterised in that** the dienophile is

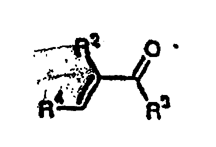

14. Process according to one of claims 10 to 13,
**characterised in that** the catalyst is (Ph₃P)₃RhCl.

15. 4-methylenespiro[2.6]nonane.

16. Use of the compound according to claim 15 or of 4-methylenespiro[2.7]decane as an odoriferous substance.

## Revendications

1. Composés de formule I 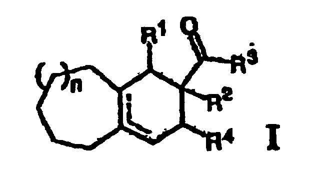 dans laquelle
n = 1,2
et R¹, R⁴ représentent H ou CH₃,
et R², R³ représentent H, CH₃ ou CH₂CH₃,
à l'exclusion du composé de formule I dans lequel
n = 1,
R¹ = R² = R⁴ = H et
R³ est CH₂CH₃.

2. Composés selon la revendication 1, correspondant à la formule I' 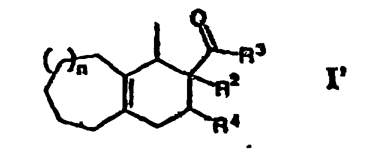 dans laquelle
n = 1,2,
et R⁴ représente H ou CH₃,
et R², R³ représentent H, CH₃ ou CH₂CH₃.

3. Composé selon la revendication 1, choisi dans le groupe constitué par la 1-{bicyclo[6.4.0]dodéc-1'(8')-èn-10'-yl}éthan-1-one (1), le 1-{bicyclo[6.4.0]-dodéc-1'(8')-èn-10'-yl}méthanal (2), la 1-{9',10'-diméthylbicyclo[6.4.0]dodéc-1'(8')-èn-10'-yl}éthan-1-one (3), la 1-{8',9'-diméthylbicyclo[5.4.0]undéc-1'(7')-èn-9'-yl}éthan-1-one (4), la 1-{9'-méthylbicyclo[6.4.0]dodéc-1'(8')-èn-10'-yl}-éthan-1-one (5), le 1-{10'-méthylbjcydo[6.4.0]dodéc-1'(8')-èn-10'-yl}méthanal (6), la 1-{11'-méthylbicyclo[6.4.0]dodéc-1'(8')-èn-10'-yl}éthan-1-one (7), la 1-{bicyclo[6.4.0]dodéc-1'(8')-èn-10'-yl}propan-1-one (8), la 1-{9',11-diméthylbicyclo[6.4.0]dodéc-1'(8')-èn-10'-yl}éthan-1-one (9), la 1-{8'-méthylbicyclo[5.4.0]undéc-l'(7')-èn-9'-yl}éthan-1-one (10), la 1-{bicyclo[6.4.0]dodéc-1'(12')-èn-10'-yl}éthan-1-one (11), le 1-{bicyclo[6.4.0]dodéc-1'(12')-èn-10'-yl}métnanal (12), la 1-{10'-méthylbicyclo[6.4.0]dodéc-1'(12')-èn-10'-yl}éthan-1-one (13) et la 1-{bicyclo[5.4.0]undéc-1'(11')-èn-9'-yl}éthan-1-one (14).

4. Composé selon la revendication 2, choisi dans le groupe constitué par la 1-{9',10'-diméthylbicyclo[6.4.0]dodéc-1'(8')-èn-10'-yl}éthan-1-one (3), la 1-{8',9'-diméthylbicyclo[5.4.0]undéc-1'(7')-èn-9'-yl}éthan-1-one (4), la 1-{9'-méthylbicyclo[6.4.0]dodéc-1'(8')-èn-10'-yl}éthan-1-one (5) et la 1-{8'-méthylbicyclo[5.4.0]undéc-1'(7')-èn-9'-yl}éthan-1-one (10).

5. Composé selon la revendication 3, choisi dans le groupe constitué par le 1-{blcyclo[6.4.0]dodéc-1'(8')-èn-10'-yl}méthanal (2) et le 1-{10'-méthylbicyclo[6.4.0]dodéc-1'(8')-èn-10'-yl}méthanal (6).

6. Composé selon la revendication 3, choisi dans le groupe constitué par le 1-(bicyclo[6.4.0]dodéc-1'(8')-èn-10'-yl}méthanal (2) et la 1-{9',10'-diméthylbicyclo[6.4.0]dodéc-1'(8')-èn-10'-yl}éthan-1-one (3).

7. Composé selon la revendication 3, choisi dans le groupe constitué par la 1-{bicyclo[6.4.0]dodéc-1'(12')-èn-10'-yl}éthan-1-one (11), le 1-{bicyclo[6.4.0]dodéc-1'(12')-èn-10'-yl}méthanal (12), la 1-{10'-méthylbicyclo[6.4.0]dodéc-1'(12')-èn-10'-yl}éthan-1-one (13) et la 1-{bicyclo[5.4.0]undéc-1'(11')-èn-9'-yl}éthan-1-one (14) en mélange avec leurs adduits de Diels-Alder régioisomères correspondants.

8. Utilisation d'un composé selon l'une quelconque des revendications 1 à 7, en tant que parfum.

9. Composition de parfum, **caractérisée en ce qu'**elle contient au moins un composé selon l'une quelconque des revendications 1 à 7.

10. Procédé pour la préparation des composés substitués par le groupe méthyle en R¹, de formule I', selon la revendication 2, **caractérisé en ce qu'**on fait réagir un composé de type vinylcyclopropane à cycle spirannique avec un diénophile, en présence d'un catalyseur régiosélectif ou Z-diastéréosélectif, pour aboutir aux composés recherchés de formule I'.

11. Procédé selon la revendication 10, **caractérisé en ce que** le composé de type vinylcyclopropane est le 4-méthylènespiro[2.6]nonane.

12. Procédé selon la revendication 10, **caractérisé en ce que** le composé de type vinylcyclopropane est le 4-méthylènespiro[2.7]décane.

13. Procédé selon l'une quelconque des revendications 10 à 12, **caractérisé en ce que** le diénophile est

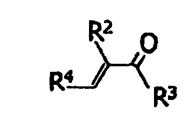

14. Procédé selon l'une quelconque des revendications 10 à 13, **caractérisé en ce que** le catalyseur est (Ph₃P)₃RhCl.

15. 4-méthylènespiro[2.6]nonane.

16. Utilisation du composé selon la revendication 15 ou du 4-méthylène-spiro[2.7]décane en tant que parfum.
